# EUROPÄISCHE PATENTSCHRIFT

(11) **EP 0 761 634 B1**
(45) Veröffentlichungstag und Bekanntmachung des Hinweises auf die Patenterteilung: **09.06.1999**
(21) Anmeldenummer: 96113606.6
(22) Anmeldetag: 24.08.1996
(51) Int. Cl.: C07C 45/49, C07C 47/21

(54) **Verfahren zur Herstellung von Pentenalen**
Process for the preparation of pentenals
Procédé de préparation de penténales

(30) Priorität: 02.09.1995 DE 19532394
(43) Veröffentlichungstag der Anmeldung: 12.03.1997
(73) Patentinhaber: Celanese GmbH, 60439 Frankfurt (DE)
(72) Erfinder: Bahrmann, Helmut, Dr., 46499 Hamminkeln (DE); Lappe, Peter, Dr., 46539 Dinslaken (DE); Wiebus, Ernst, 46147 Oberhausen (DE); Fell, Bernhard, Prof. Dr., 52074 Aachen (DE); Hermanns, Peter, 52078 Aachen (DE)

(56) Entgegenhaltungen:
- EP-A- 0 033 554
- EP-A- 0 562 450
- EP-A- 0 643 031
- DE-A- 2 627 354

## Beschreibung

Die Erfindung betrifft ein Verfahren zur Herstellung von Pentenalen durch Hydroformylierung von 1,3-Butadien, das in einem unter Reaktionsbedingungen inerten Medium eingesetzt wird. Die Reaktion erfolgt in Gegenwart einer wäßrigen Katalysatorphase, die eine Rhodium/Phosphin-Komplexverbindung und gegebenenfalls noch weiteres Phosphin als Katalysator enthält.

1,3-Butadien ist als Nebenprodukt der Rohölverarbeitung, es fällt z.B. bei der Naphtha-Pyrolyse als Bestandteil der C₄-Fraktion an, ein in großen Mengen verfügbarer Rohstoff für chemische Synthesen. Ein Weg zu seiner Nutzung ist die Umsetzung mit Kohlenmonoxid und Wasserstoff (Hydroformylierung) zu Carbonyl- und durch Weiterreaktion zu Hydroxymethylverbindungen. Die Hydroformylierung von 1,3-Butadien ist vielfach untersucht worden. Bedingt durch das Vorhandensein von zwei zueinander konjugierten Doppelbindungen sind als Reaktionsprodukte nicht nur Dialdehyde und Monoaldehyde in ihren verschieden strukturisomeren Formen zu erwarten, sondern auch ungesättigte C₅-Aldehyde.

Dialdehyde bilden sich nach B. Fell et al. bei der Hydroformylierung von 1,3-Butadien in Gegenwart phosphin-modifizierter Rhodiumkatalystoren (vgl. Chem.-Ztg. 99 (1975), 485 ff). Die gesättigten C₅-Monoaldehyde werden als Reaktionsprodukte bei der Hydroformylierung in Gegenwart von Kobaltcarbonyl-, Rhodiumcarbonyl-oder durch Phosphine modifizierte Rhodiumcarbonyl-Katalysatoren erhalten (vgl. B. Fell et al. Forschungsbericht Bundesministerium Forschung und Technologie, BMFT-FB-T-84 064, Karlsruhe 1984).

Im Gegensatz zur Synthese von C₅-Aldehyden und C₆-Dialdehyden durch Reaktion von Butadien mit Kohlenmonoxid und Wasserstoff verläuft die Hydroformylierung des 1,3-Diolefins zu ungesättigten gesättigten C₅-Aldehyden mit nur geringer Selektivität. Bei der Umsetzung der Reaktanten im homogenen Reaktionssystem mit Rhodiumcarbonyl/Phosphin-Katalysatoren bilden sich auch unter wechselnden Reaktionsbedingungen bevorzugt Pentanale. Die Hydroformylierung von 1,3-Butadien in Gegenwart wasserlöslicher Rhodiumcarbonyl/Phosphin-Katalysatoren ergibt zwar primär n-Pentenal-3 als Reaktionsprodukt, der hochreaktive ungesättigte Aldehyd wird jedoch durch Aldolkondensation in das 2-Propenylheptadienal und weiter in C₁₅- und höhermolekulare Aldolprodukte überführt.

So offenbart EP-A-0 643 031 ein Verfahren zur Hydroformylierung von 1,3-Butadien in Gegenwart einer wäßrigen, eine wasserlösliche Rhodiumverbindung und mindestens ein wasserlösliches organisches Phosphin enthaltende Lösung, indem reines 1,3-Butadien ohne Zusatz eines Lösungsmittels eingesetzt wird. Bei diesem Verfahren reagieren die zunächst gebildeten C₅-Aldehyde teilweise zu ungesättigten Folgeprodukten weiter, z.B. zu ungesättigten C₁₀-Aldehyden, die zu 2-Propylheptanol weiterhydriert werden können.

Ebenfalls beschreibt EP-A-0 562 450 ein Verfahren zur Hydroformylierung von 1,3-Butadien in Gegenwart wasserlöslicher Rhodiumkatalysatoren mit dem Ziel, eine möglichst hohe Selektivität an gesättigtem n-Valeraldehyd zu erhalten. Nach EP-A-0 562 450 wird lediglich ein Teil des 1,3-Butadiens in Aldehyd überführt und der Rest zirkuliert. Das beschriebene Verfahren verwendet 1,3-Butadien ohne Lösungsmittelzusatz in einer Reinheit von mindestens 99,5 Gew.-%.

Im Hinblick darauf, daß ungesättigte C₅-Aldehyde wegen des Vorliegens der Aldehydfunktion und einer Doppelbindung im Molekül als Zwischenprodukte die zahlreichen Reaktionen zugänglich sind, großes technisches Interesse besitzen, bestand die Aufgabe, ein Verfahren zu entwickeln, das die Herstellung von Pentenalen mit hoher Selektivität und Ausbeute erlaubt.

Die Erfindung besteht in einem Verfahren zur Herstellung von Pentenalen durch Hydroformylierung von 1,3-Butadien. Es ist dadurch gekennzeichnet, daß man das 1,3-Butadien gelöst in einem unter Reaktionsbedingungen inerten Medium einsetzt, für das man aliphatische oder cycloaliphatische Kohlenwasserstoffe mit 3 bis 10 Kohlenstoffatomen im Molekül oder aromatische Kohlenwasserstoffe mit 6 bis 9 Kohlenstoffatomen im Molekül oder Ether verwendet und die Hydroformylierung bei 100 bis 180° und 5 bis 35 MPa in Gegenwart einer wäßrigen Lösung, die eine Rhodium/Phosphin-Komplexverbindung und gegebenenfalls noch weiteres Phosphin als Katalysator, enthält, umsetzt.

Überraschenderweise hat sich gezeigt, daß die Selektivität der Umwandlung von 1,3-Butadien durch Hydroformylierung in einem heterogenen Reaktionssystem erheblich verbessert wird, wenn das 1,3-Butadien nicht als reiner Stoff, sondern in einem Medium gelöst eingesetzt wird, das unter den Reaktionsbedingungen inert ist. Die Verdünnung des Diolefins führt insbesondere dazu, daß die Folgereaktionen, Bildung von Aldolkondensationsprodukten, vermieden werden. Als inerte Medien eignen sich gesättigte aliphatische und cycloaliphatische Kohlenwaserstoffe mit 3 bis 10 Kohlenstoffatomen im Molekül sowie aromatische Kohlenwasserstoffe mit 6 bis 9 Kohlenstoffatomen im Molekül. Beispiele für die genannten Verbindungsklassen sind Propan, n-Butan, n-Hexan, Cyclohexan, Toluol und die Xylole. Mit gleich gutem Erfolg wie aliphatische oder aromatische Kohlenwasserstoffe werden auch Ether als Lösungsmittel für das 1,3-Butadien verwendet. Sie werden beispielhaft durch die Verbindungen Diethylether oder Methyl-tert.-butylether vertreten.

Es hat sich als zweckmäßig erwiesen, daß ein Volumenteil 1,3-Butadien in mindestens einem Volumenteil des inerten Mediums gelöst ist. Im allgemeinen löst man ein Volumenteil 1,3-Butadien in ein bis zehn Volumenteilen des inerten Mediums.

Eine besonders zweckmäßige Ausgestaltung des erfindungsgemäßen Verfahrens besteht darin, 1,3-Butadien in Form technischer Kohlenwasserstoffgemische einzusetzen. Solche Gemische fallen z.B. als Raffinerie-Nebenprodukte bei der Herstellung von Automobiltreibstoff und bei der Herstellung von Ethylen durch thermische Spaltung höherer Kohlenwasserstoffe zwangsweise in erheblichen Mengen an (C₄-Crackschnitte). Neben 1,3-Butadien und gesättigten C₄-Kohlenwasserstoffen enthalten sie auch n-Buten-1, Isobuten und cis- und trans-n-Buten-2. Unter den Bedingungen der Butadien-Hydroformylierung verhalten sich die genannten Olefine, ausgenommen n-Buten-1, weitgehend inert und bilden zusammen mit den gesättigten C₄-Kohlenwasserstoffen n- und i-Butan, das inerte Medium, in dem erfindungsgemäß das 1,3-Butadien gelöst ist.

Die Umsetzung 1,3-Butadien enthaltender technischer Kohlenwasserstoffgemische nach dem erfindungsgemäßen Verfahren erlaubt somit nicht nur die Herstellung olefinisch ungesättigter C₅-Aldehyde, sondern führt im Sinne eines neuartigen Carbozu einem Raffinat, das aus Isobuten, cis- und trans-n-Buten-2 und den gesättigten C₄-Kohlenwasserstoffen besteht und 1,3-Butadien sowie n-Buten-1 nur noch in Spuren enthält. Aus diesem Gemisch kann Isobuten nach bekannten Verfahren, z.B. durch Umwandlung in Methyl-tert.-butylether abgetrennt werden. Die verbleibenden C₄-Kohlenwasserstoffe werden entweder in den Pyrolyseprozeß zurückgeführt oder in anderer Weise chemisch umgesetzt.

Die Hydroformylierung des in dem inerten Medium gelösten 1,3-Butadiens führt man als heterogene Reaktion in einem Zweiphasensystem durch, eine Umsetzung, die z.B. in der DE-PS 26 27 354 beschrieben ist. Dieser Prozeß ist charakterisiert durch das Vorliegen einer organischen Phase, die u.a. die olefinische Verbindung und das Reaktionsprodukt enthält und einer wäßrigen Phase, in der der Katalysator gelöst ist. Als Katalysatoren werden wasserlösliche Rhodiumkomplexverbindungen eingesetzt, die wasserlösliche organische Phosphine als Liganden enthalten. Üblicherweise liegt das wasserlösliche organische Phosphin, bezogen auf Rhodium, im Überschuß vor, d.h. im Katalysatorsystem ist neben der Rhodium/Phosphin-Komplexverbindung auch noch freies Phosphin vorhanden.

Unter dem Begriff wasserlösliche organische Phosphine werden Mono- oder Polyphosphine verstanden, in denen an die Valenzen des dreiwertigen Phosphoratoms oder der dreiwertigen Phosphoratome Alkyl- und/oder Arylgruppen gebunden sind, von denen mindestens eine einfach oder mehrfach sulfoniert oder carboxyliert ist. Die Herstellung solcher Phosphine ist bekannt und z.B. in DE-PS 26 27 354 und DD-PS 259 194 beschrieben.

Als wasserlösliche organische Phosphine besonders bewährt haben sich Verbindungen der allgemeinen Formel (1)

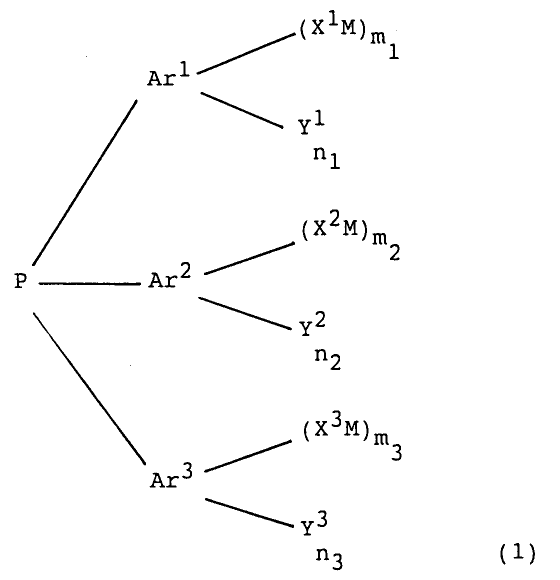

Hierbei bedeuten Ar¹, Ar², Ar³ jeweils eine Phenyl- oder Naphthylgruppe. X¹, X², X³ ist jeweils ein Sulfonat-(-SO₃⁻) und/oder Carboxylat-(-COO⁻)-Rest, Y¹, Y², Y³ ist jeweils eine geradkettige oder verzweigte Alkylgruppe mit 1 bis 4 C-Atomen, eine Alkoxygruppe, ein Halogenatom, die OH-, CN-, NO₂- oder (R¹R²)N-Gruppe, in der R¹ und R² für jeweils eine geradkettige oder verzweigte Alkylgruppe mit 1 bis 4 C-Atomen stehen. m₁, m₂, m₃ sind gleiche oder verschiedene ganze Zahlen von 0 bis 5 mit der Maßgabe, daß mindestens ein m₁, m₂ oder m₃ größer als 0 ist. n₁, n₂, n₃ sind gleiche oder verschiedene ganze Zahlen von 0 bis 5. M ist ein Alkalimetallion, ein chemisches Äquivalent eines Erdalkalimetall- oder Zinkions, ein Ammonium- oder quaternäres Ammoniumion der allgemeinen Formel N(R³R⁴R⁵R⁶)⁺ in der R³, R⁴, R⁵, R⁶ gleich oder verschieden sind und für Wasserstoff und für geradkettige oder verzweigte Alkylgruppe mit 1 bis 4 C-Atomen stehen.

Besonders bewährt haben sich Phosphine der oben wiedergegebenen Formel (1), in der Ar¹, Ar², Ar³ jeweils einen Phenylrest und X¹, X², X³ jeweils einen in meta-Stellung zum Phosphor befindlichen Sulfonatrest bedeuten, d.h. Salze des Tris-(m-sulfonatophenyl)phosphins (TPPTS).

Die in den Katalysatorsystemen enthaltenen Phosphine brauchen nicht einheitliche chemische Verbindungen zu sein, sondern können unterschiedliche chemische Zusammensetzung aufweisen. So können sie sich z.B. durch Art und Bindung der am Phosphoratom haftenden Reste, durch den Sulfonierungs- oder Carboxylierungsgrad oder durch die Art der Kationen unterscheiden.

Das Katalysatorsystem kann dem Reaktionsgemisch präformiert zugesetzt werden. Mit gleich gutem Erfolg läßt es sich aber auch aus den Komponenten Rhodium oder Rhodiumverbindung und dem Phosphin unter Reaktionsbedingungen im Reaktionsgemisch herstellen. Außer metallischem Rhodium in feinverteilter Form können als Rhodiumquelle Rhodiumsalze wie Rhodium(III)-sulfat, Rhodium(III)-nitrat, Rhodium(II)-acetat, Rhodiumacetylacetonat, Rhodiumoxide oder andere carbonylbildende Verbindungen des Rhodiums eingesetzt werden.

Die Rhodiumkonzentration in der wäßrigen Katalysatorphase beträgt 100 bis 600 Gew.-ppm, vorzugsweise 300 bis 500 Gew.-ppm, bezogen auf die Lösung. Üblicherweise wird das Phosphin in einer solchen Menge eingesetzt, daß je mol Rhodium mindestens 20 mol, vorzugsweise 40 bis 80 mol P(III) vorliegen. Die Umsetzung des 1,3-Butadiens mit Wasserstoff und Kohlenmonoxid erfolgt bei Temperaturen von 100 bis 180°C, vorzugsweise 110 bis 120°C und Drücken von 5 bis 35 MPa, vorzugsweise 4 bis 8 MPa.

Der pH-Wert der Katalysatorlösung beträgt 4 bis 10, vorzugsweise 5,5 bis 9.

Das Verhältnis von Kohlenmonoxid zu Wasserstoff im Synthesegas kann in weiten Grenzen variiert werden. Im allgemeinen setzt man Synthesegas ein, in dem das Volumverhältnis von Kohlenmonoxid zu Wasserstoff 1 : 1 beträgt oder von diesem Wert nur wenig abweicht.

Das Volumverhältnis von Katalysatorphase zu organischer Phase kann im Bereich von 1 : 4 bis 4 : 1 variiert werden, vorteilhaft ist ein Verhältnis von 1 : 1 bis 2 : 1.

Die Umsetzung kann sowohl absatzweise als auch kontinuierlich durchgeführt werden.

Zur Aufarbeitung des Reaktionsproduktes werden organische Phase und wäßrige Katalysatorphase voneinander getrennt. Aus der Katalysatorphase werden die Hydroformylierungsprodukte destillativ isoliert.

Das erfindungsgemäße Verfahren wird durch die nachfolgenden Beispiele erläutert, jedoch nicht auf die beschriebenen Ausführungsformen beschränkt.

### Beispiel 1 (Vergleichsbeispiel)

In einem 160 ml V4A-Edelstahlautoklav mit druckfestem Dosiergefäß, Druckaufnehmer, Berstscheibe, Thermoelement und einem magnetgekuppelten Propellerrührer wurden in Argonatmosphäre 40 g einer wäßrigen Katalysatorlösung mit 16,4 mg Rhodium (in Form einer wäßrigen Rh(III)-sulfatlösung), entsprechend 410 ppm Rh in der Lösung, sowie 3,77 g Na₃-TPPTS vorgelegt. Der pH-Wert wurde mittels Na₂CO₃ auf einen Wert von 6 eingestellt und der Autoklav verschlossen. Danach wurden 20 g 1,3-Butadien aus einer Druckgasflasche in eine mit zwei Ventilen versehene und evakuierte Metallkapillare einkondensiert. Als innerer Standard für die gaschromatographische Analyse (GC-Analyse) wurde zusätzlich noch eine abgewogene Menge Propan zugegeben. Die Metallwendel mit der 1,3-Butadien/Propan-Probe wurde auf das Dosiergefäß geschraubt, die Katalysatorlösung nach Spülen des Autoklavs mit Wassergas (CO/H₂ = 1/1) 90 Minuten lang bei 110°C und 10 MPa präformiert und dann im Autoklav der gewünschte Reaktionsdruck für die Hydroformylierung von 6 MPa eingestellt. Das in der Metallwendel befindliche Flüssiggas wurde in den intensiv gerührten Autoklav gepreßt und dadurch die Reaktion in Gang gebracht. Nach einer Reaktionszeit von 5 h wurde abgekühlt, von der Gasphase zur Umsatzbestimmung Menge und Zusammensetzung bestimmt und die wäßrige Katalysatorphase nach Abtrennung des organischen Reaktionsproduktes noch dreimal mit je 15 ml Diethylether extrahiert. Die Lösung des Reaktions-produktes im Ether wurde über Na₂SO₄ getrocknet und darauf gas-chromatographisch analysiert.

| | | | |
|---|---|---|---|
| Ergebnis: | Umsatz: | 89 % | |
| | Zusammensetzung: | n-Pentenale/n-Pentanal | 34 % |
| | | 2-Propenylheptadienal | 34 % |
| | | 2-Methylbutanal | 2 % |
| | | Sonstige Produkte*⁾ | 11 % |
| | | Dicköl (> C₁₀) | 19 % |

| | | | |
|---|---|---|---|
| *) Es handelt sich hauptsächlich um Formylcyclopenten, dem intramolekularen Aldolkondensationsprodukt von 1,6-Hexandial, das durch Hydroformylierung von n-Pentenal-4 gebildet wird. In sehr geringen Mengen werden als weitere Bis-hydroformylierungsprodukte auch 2-Methylpentandial und Ethylbutandial gebildet. | | | |

### Beispiel 2

Unter den Bedingungen des Beispiels 1, jedoch bei auf 4 h verkürzter Reaktionszeit, wurde 1,3-Butadien in Gegenwart von Diethylether als Lösungsmittel (Volumverhältnis Lösungsmittel/Butadien = 2) umgesetzt.

| | | | |
|---|---|---|---|
| Ergebnis: | Umsatz: | 82 % | |
| | Zusammensetzung: | n-Pentenale/n-Pentanal | 85 % |
| | | 2-Propenylheptadienal | 1,5 % |
| | | 2-Methylbutanal | 1,5 % |
| | | Sonstige Produkte | 10 % |
| | | Dicköl (> C₁₀) | 2 % |

Zusammensetzung der n-Pentenale/n-Pentanal-Fraktion: 72 % cis/trans 3-Pentenal, 3 % 4-Pentenal, 1 % 2-Pentenal und 23 % n-Pentanal.

Die in den folgenden Beispielen 3 bis 12 beschriebenen Versuche wurden mit einer durch Pyrolyse von Naphtha erhaltenen C₄-Fraktion der nachstehenden Zusammensetzung durchgeführt:

| | |
|---|---|
| 1,3-Butadien | 46 % |
| i-Buten | 23,5 % |
| n-Buten-1 | 11,5 % |
| trans-n-Buten-2 | 5 % |
| cis-n-Buten-2 | 4 % |
| 1,2-Butadien, Butenin, Butine | 1 % |
| n-Butan | 6 % |
| i-Butan | 3 % |

### Beispiele 3 bis 6

In den Beispielen 3 bis 6 wurden die Reaktionstemperatur und die Reaktionszeit variiert, der Ansatz (20 g C₄-Fraktion) und die übrigen Reaktionsbedingungen stimmten mit denen des Beispiels 1 überein.

### Beispiele 3 bis 6: Zusammensetzung der Reaktionsprodukte (Gew.-%)

| Beispiel | Reaktionstemp. -zeit | | n-Pentenale/n-Pentanal* | 2-Propenylheptadienal | 2- | 3- | Formylcyclopenten | Dicköl Rest** (>C10) |
|---|---|---|---|---|---|---|---|---|
| | °C | h | | | Methylbutanal | | | |
| 3 | 100 | 6 | 67 (49) | 6 | 3 | 1 | 6 | 5 |
| 4 | 110 | 4 | 68 (62) | 9 | 4 | 1 | 7 | 2 |
| 5 | 120 | 2 | 69 (73) | 6 | 4 | 1 | 6 | 3 |
| 6 | 120 | 12 | 57 (18) | 10 | 5 | 6 | 8 | 2 |

| | | | | | | | | |
|---|---|---|---|---|---|---|---|---|
| * In Klammern der Anteil des cis/trans-n-Pentenal-3 an der C₅-Fraktion | | | | | | | | |
| ** Der Rest bis 100 % besteht aus verschiedenen nichtidentifizierten Verbindungen, die weniger als 10 C-Atome im Molekül enthalten. | | | | | | | | |

### Beispiele 3 bis 6: Umsatz der C₄-Bestandteile des Einsatzmaterials (Gew.-%)

| Beispiel | 1,3-Butadien | n-Buten-1 | cis- | trans- | i-Buten |
|---|---|---|---|---|---|
| | | | n-Buten-2 | | |
| 3 | 90 | 63 | 10 | 7 | 11 |
| 4 | 91 | 79 | 5 | <1 | 11 |
| 5 | 91 | 69 | 1 | 0 | 9 |
| 6 | 94 | 93 | 23 | 5 | 24 |

### Beispiele 7 bis 9

In den Beispielen 7 bis 9 erfolgte die Hydroformylierung der C₄-Fraktion unter Variation des P/Rh-Verhältnisses in der wäßrigen Katalysatorlösung. Die übrigen Bedingungen stimmten bis auf den Reaktionszeit, sie betrug 4 h, mit denen des Beispiels 1 überein.

### Beispiele 7 bis 9: Zusammensetzung der Reaktionsprodukte (Gew.-%)

| Beispiel | P/Rh-Verhältnis | n-Pentenale/n-Pentanal* | 2-Propenylheptadienal | 2- | 3- | Formylcyclopenten | Dicköl Rest** (> C10) |
|---|---|---|---|---|---|---|---|
| | | | | Methylbutanal | | | |
| 7 | 10 | 63 (66) | 6 | 4 | <1 | 7 | 9 |
| 8 | 40 | 68 (62) | 9 | 4 | 1 | 7 | 2 |
| 9 | 60 | 68 (59) | 10 | 3 | 1 | 6 | 3 |

| | | | | | | | |
|---|---|---|---|---|---|---|---|
| * In Klammern der Anteil des cis/trans-n-Pentenal-3 an der C₅-Fraktion | | | | | | | |
| ** Der Rest bis 100 % besteht aus verschiedenen nichtidentifizierten Verbindungen, die weniger als 10 C-Atome im Molekül enthalten. | | | | | | | |

### Beispiele 7 bis 9: Umsatz der C₄-Bestandteile des Einsatzmaterials (Gew.-%)

| Beispiel | 1,3-Butadien | n-Buten-1 | cis- | trans- | Isobuten |
|---|---|---|---|---|---|
| | | | n-Buten-2 | | |
| 7 | 80 | 34 | 1 | 0 | 5 |
| 8 | 92 | 79 | 5 | 0 | 11 |
| 9 | 91 | 68 | 12 | 9 | 19 |

### Beispiele 10 bis 12

Aus den Beispielen 10 bis 12 geht der Einfluß des pH-Wertes der wäßrigen Katalysatorphase auf die Hydroformylierung der C₄-Fraktion hervor. Es wurde wiederum unter den in Beispiel 1 angegebenen Reaktionsbedingungen, jedoch bei auf 4 h verkürzter Reaktionszeit gearbeitet.

### Beispiele 10 bis 12: Zusammensetzung der Reaktionsprodukte (Gew.-%)

| Beispiel | pH-Wert | n-Pentenale/n-Pentanal | 2-Propenylheptadienal | 2- | 3- | Formylcyclopenten | Dicköl Rest** (> C10) |
|---|---|---|---|---|---|---|---|
| | | | | Methylbutanal | | | |
| 10 | 5 | 81(65) | 2 | 3 | 1 | 6 | <1 |
| 11 | 7 | 74(64) | 5 | 3 | 1 | 7 | 3 |
| 12 | 9 | 68(62) | 9 | 4 | 1 | 7 | 2 |

| | | | | | | | |
|---|---|---|---|---|---|---|---|
| * In Klammern der Anteil an cis/trans-Pentenal-3 an der C₅-Fraktion | | | | | | | |
| ** Der Rest bis 100 % besteht aus verschiedenen nichtidentifizierten Verbindungen, die weniger als 10 C-Atome im Molekül enthalten. | | | | | | | |

## Patentansprüche

1. Verfahren zur Herstellung von Pentenalen durch Hydroformylierung von 1,3-Butadien, dadurch gekennzeichnet, daß man das 1,3-Butadien gelöst in einem unter Reaktionsbedingungen inerten Medium einsetzt, für das man aliphatische oder cycloaliphatische Kohlenwasserstoffe mit 3 bis 10 Kohlenstoffatomen im Molekül oder aromatische Kohlenwasserstoffe mit 6 bis 9 Kohlenstoffatomen im Molekül oder Ether verwendet und die Hydroformylierung bei 100 bis 180°C und 5 bis 35 MPa in Gegenwart einer wäßrigen Lösung, die eine Rhodium/Phosphin-Komplexverbindung und gegebenfalls noch weiteres Phosphin als Katalysator enthält, umsetzt.

2. Verfahren nach Anspruch 1, dadurch gekennzeichnet, daß die Lösung von 1,3-Butadien in einem inerten Medium ein bei der Verarbeitung von Rohöl anfallendes Gemisch von C₄-Kohlenwasserstoffen ist.

3. Verfahren nach Anspruch 1 oder 2, dadurch gekennzeichnet, daß 1 Volumteil des 1,3-Butadiens in mindestens 1 Volumteil des inerten Mediums gelöst ist.

4. Verfahren nach Anspruch 3, dadurch gekennzeichnet, daß 1 Volumteil des 1,3-Butadiens in 1 bis 10 Volumteilen des inerten Mediums gelöst ist.

5. Verfahren nach einem oder mehreren der Ansprüche 1 bis 4, dadurch gekennzeichnet, daß die Rhodium/Phosphin-Komplexverbindung ein Monophosphin der allgemeinen Formel 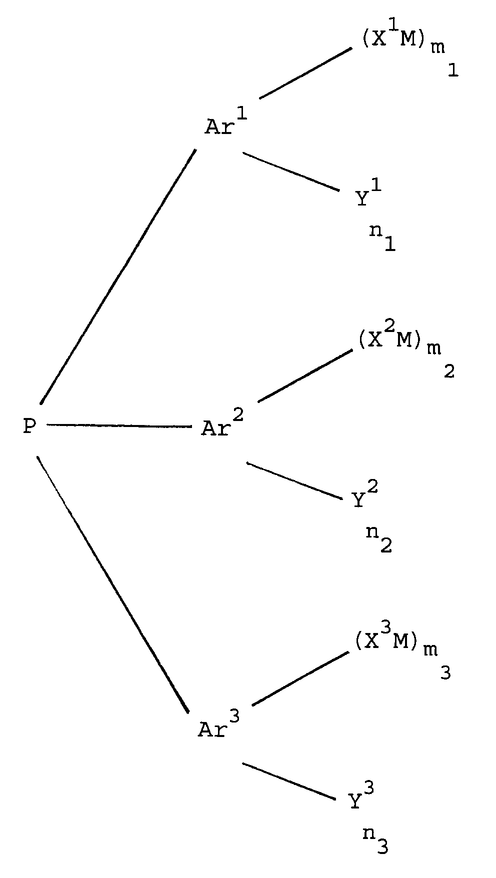 enthält, in der Ar¹, Ar², Ar³ jeweils eine Phenyl- oder Naphthylgruppe bedeutet, X¹, X², X³ jeweils ein Sulfonat-(-SO₃⁻) und/oder Carboxylat-(-COO⁻)-Rest ist, Y¹, Y², Y³ jeweils für eine geradkettige oder verzweigte Alkylgruppe mit 1 bis 4 C-Atomen, eine Alkoxygruppe, ein Halogenatom, die OH-, CN-, NO₂- oder (R¹R²)-N-Gruppen, in der R¹ und R² jeweils eine geradkettige oder verzweigte Alkylgruppe mit 1 bis 4 C-Atomen sind, steht, m₁, m₂, m₃ gleiche oder verschiedene ganze Zahlen von 0 bis 5 sind mit der Maßgabe, daß mindestens ein m₁, m₂ oder m₃ größer als 0 ist, n₁, n₂, n₃ gleiche oder verschiedene ganze Zahlen von 0 bis 5 sind, M ein Alkalimetallion, ein chemisches Äquivalent eines Erdalkalimetall- oder Zinkions, ein Ammonium- oder quaternäres Ammoniumion der allgemeinen Formel N(R³R⁴R⁵R⁶)⁺ in der R³, R⁴, R⁵, R⁶ gleich oder verschieden sind und für Wasserstoff und für geradkettige oder verzweigte Alkylgruppen mit 1 bis 4 C-Atomen stehen, ist.

6. Verfahren nach Anspruch 5, dadurch gekennzeichnet, daß die Rhodium/Phosphin-Komplexverbindung ein Tris(m-sulfonatophenyl)phosphin-Salz enthält.

7. Verfahren nach einem oder mehreren der Ansprüche 1 bis 6, dadurch gekennzeichnet, daß die Umsetzung bei 110 bis 120°C und 4 bis 8 MPa erfolgt.

8. Verfahren nach einem oder mehreren der Ansprüche 1 bis 7, dadurch gekennzeichnet, daß die Rhodiumkonzentration in der wäßrigen Katalysatorlösung 100 bis 600 Gew.-ppm, bezogen auf die Lösung, beträgt.

9. Verfahren nach Anspruch 8, dadurch gekennzeichnet, daß die Rhodiumkonzentration in der wäßrigen Katalysatorlösung 300 bis 500 Gew.-ppm, bezogen auf die Lösung, beträgt.

10. Verfahren nach einem oder mehreren der Ansprüche 1 bis 9, dadurch gekennzeichnet, daß je mol Rhodium mindestens 20 mol P(III) in Form eines Phosphins vorliegen.

11. Verfahren nach Anspruch 10, dadurch gekennzeichnet, daß je mol Rhodium 40 bis 80 mol P(III) in Form eines Phosphins vorliegen.

12. Verfahren nach einem oder mehreren der Ansprüche 1 bis 11, dadurch gekennzeichnet, daß der pH-Wert der wäßrigen Katalysatorlösung 4 bis 10 beträgt.

13. Verfahren nach Anspruch 12, dadurch gekennzeichnet, daß der pH-Wert der wäßrigen Katalystorlösung 5,5 bis 9 beträgt.

14. Verfahren nach einem oder mehreren der Ansprüche 1 bis 13, dadurch gekennzeichnet, daß das Volumverhältnis von wäßriger zu organischer Phase 1 : 4 bis 4 : 1 beträgt.

15. Verfahren nach Anspruch 14, dadurch gekennzeichnet, daß das Volumverhältnis von wäßriger zu organischer Phase 1 : 1 bis 1 : 2 beträgt.

## Claims

1. A process for preparing pentenals by hydroformylation of 1,3 -butadiene, characterised in that the 1,3-butadiene is used dissolved in a medium which is inert under the reaction conditions, aliphatic or cycloaliphatic hydrocarbons having 3 to 10 carbon atoms in the molecule or aromatic hydrocarbons having 6 to 9 carbon atoms in the molecule or ether being used as the inert medium, and the hydroformylation is carried out at 100 to 180°C and 5 to 35 MPa in the presence of an aqueous solution comprising a rhodium/phosphine complex and, optionally, a further phosphine as catalyst.

2. The process according to claim 1, characterised in that the solution of 1,3-butadiene in an inert medium is a mixture of C ₄-hydrocarbons obtained in the refining of crude oil.

3. The process according to claim 1 or 2, characterised in that 1 part by volume of the 1,3-butadiene is dissolved in at least 1 part by volume of the inert medium.

4. The process according to claim 3, characterised in that 1 part by volume of the 1,3-butadiene is dissolved in 1 to 10 parts by volume of the inert medium.

5. The process according to one or more of claims 1 through 4, characterised in that the rhodium/phosphine complex contains a monophosphine of the formula 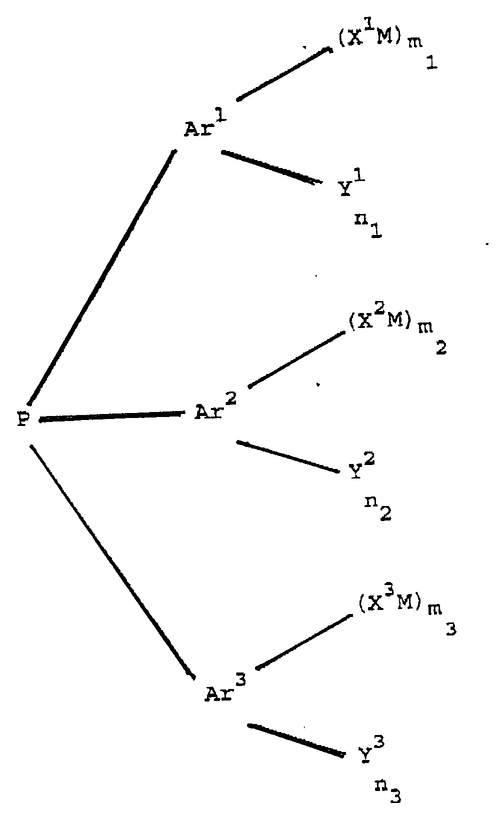 where Ar¹, Ar², Ar³ are each a phenyl or naphthyl group, X¹, X², X³ are each a sulfonate(-SO₃⁻) and/or carboxylate (-COO⁻) radical, Y¹, Y², Y³ are each a straight-chain or branched alkyl group having 1 to 4 carbon atoms, an alkoxy group, a halogen atom, the OH, CN, NO₂ or (R¹R²)-N group in which R¹ and R² are each a straight-chain or branched alkyl group having 1 to 4 carbon atoms, m₁, m₂, m₃ are identical or different integers from 0 to 5, with the proviso that at least one m₁, m₂, m₃ is greater than 0, n₁, n₂, n₃ are identical or different integers from 0 to 5, M is an alkaline earth metal ion, a chemical equivalent of an alkaline earth metal or zinc ion, an ammonium or quaternary ammonium ion of the formula N(R³R⁴ R⁵R⁶)⁺ in which R³, R⁴, R⁵, R⁶ stand for identical or different and are hydrogen or straight-chain or branched alkyl groups having 1 to 4 carbon atoms

6. The process according to claim 5, characterised in that the rhodium/phosphine complex contains tris(m-sulfonatophenyl)phosphine salt.

7. The process according to one or more of claims 1 through 6, characterised in that the reaction is carried out at 110 to 120°C and 4 to 8 MPa.

8. The process according to one or more of claims 1 through 7, characterised in that the rhodium concentration in the aqueous catalyst solution is 100 to 600 ppm by weight, related to the solution

9. The process according to claim 8, characterised in that the rhodium concentration in the aqueous catalyst solution is 300 to 500 ppm by weight, related to the solution

10. The process according to one or more of claims 1 through 9, characterised in that at least 20 mol of P(III) in the form of a phosphine are present per mole of rhodium.

11. The process according to claim 10, characterised in that 40 to 80 mol of P(III) in the form of a phosphine are present per mole of rhodium.

12. The process according to one or more of claims 1 through 11, characterised in that the pH of the aqueous catalyst solution is 4 to 10.

13. The process according to claim 12, characterised in that the pH of the aqueous catalyst solution is 5.5 to 9.

14. The process according to one or more of claims 1 through 13, characterised in that the volume ratio of aqueous to organic phase is 1 : 4 to 4 : 1.

15. The process according to claim 14, characterised in that the volume ratio of aqueous to organic phase is 1 : 1 to 1 : 2.

## Revendications

1. Procédé de préparation de penténals par hydroformylation de 1,3-butadiène, caractérisé en ce que l'on dissout le 1,3-butadiène dans un milieu inerte dans les conditions de la réaction, pour lequel on utilise des hydrocarbures aliphatiques ou cycloaliphatiques ayant 3 à 10 atomes de carbone dans la molécule ou des hydrocarbures aromatiques ayant 6 à 9 atomes de carbone dans la molécule ou des éthers, et on effectue l'hydroformylation à une température de 100 à 180°C et sous 5 à 35 MPa en présence d'une solution aqueuse contenant comme catalyseur un composé complexe de rhodium/phosphine et éventuellement une quantité supplémentaire de phosphine.

2. Procédé selon la revendication 1, caractérisé en ce que la solution de 1,3-butadiène dans un milieu inerte est un mélange d'hydrocarbures en C₄ se formant lors du traitement de pétrole brut.

3. Procédé selon la revendication 1 ou 2, caractérisé en ce que 1 partie en volume de 1,3-butadiène est dissoute dans au moins 1 partie en volume de milieu inerte.

4. Procédé selon la revendication 3, caractérisé en ce que 1 partie en volume de 1,3-butadiène est dissoute dans 1 à 10 parties en volume de milieu inerte.

5. Procédé selon l'une ou plusieurs des revendications 1 à 4, caractérisé en ce que le composé complexe de rhodium/phosphine contient une monophosphine de formule générale 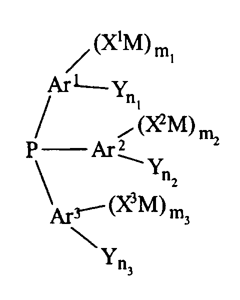 dans laquelle Ar¹, Ar², Ar³ représentent chacun un groupe phényle ou naphtyle; X¹, X², X³ sont chacun un reste sulfonate (-SO₃⁻) et/ou carboxylate (-COO⁻); Y¹, Y², Y³ sont chacun un groupe alkyle linéaire ou ramifié de 1 à 4 atomes de carbone, un groupe alcoxy, un atome d'halogène, un groupe OH, CN, NO₂ ou (R¹R¹)N, où R¹ et R² sont chacun un groupe alkyle linéaire ou ramifié de 1 à 4 atomes de carbone; m₁, m₂, m₃ sont des nombres entiers identiques ou différents de 0 à 5, à condition qu'au moins l'un des m₁, m₂, m₃ soit supérieur à 0; n₁, n₂, n₃ sont des nombres entiers identiques ou différents de 0 à 5; M est un ion de métal alcalin, un équivalent chimique d'un ion de métal alcalino-terreux ou de zinc, un ion ammonium ou ammonium quaternaire de formule générale N(R³R⁴R⁵R⁶)⁺ dans laquelle R³, R⁴, R⁵, R⁶ sont identiques ou différents et représentent des atomes d'hydrogène ou des groupes alkyle linéaires ou ramifiés de 1 à 4 atomes de carbone.

6. Procédé selon la revendication 5, caractérisé en ce que le composé complexe de rhodium/phosphine contient un sel de tris(m-sulfonatophényl)phosphine.

7. Procédé selon l'une ou plusieurs des revendications 1 à 6, caractérisé en ce que la réaction s'effectue à une température de 110 à 120°C et sous 4 à 8 MPa.

8. Procédé selon l'une ou plusieurs des revendications 1 à 7, caractérisé en ce que la concentration de rhodium dans la solution aqueuse de catalyseur est comprise entre 100 et 600 ppm en masse par rapport à la solution.

9. Procédé selon la revendication 8, caractérisé en ce que la concentration de rhodium dans la solution aqueuse de catalyseur est comprise entre 300 et 500 ppm en masse par rapport à la solution.

10. Procédé selon l'une ou plusieurs des revendications 1 à 9, caractérisé en ce qu'il y a au moins 20 mol de P(III) sous forme de phosphine par mol de rhodium.

11. Procédé selon la revendication 10, caractérisé en ce qu'il y a 40 à 80 mol de P(III) sous forme de phosphine par mol de rhodium.

12. Procédé selon l'une ou plusieurs des revendications 1 à 11, caractérisé en ce que le pH de la solution aqueuse de catalyseur est compris entre 4 et 10.

13. Procédé selon la revendication 12, caractérisé en ce que le pH de la solution aqueuse de catalyseur est compris entre 5,5 et 9.

14. Procédé selon l'une ou plusieurs des revendications 1 à 13, caractérisé en ce que le rapport en volume de la phase aqueuse à la phase organique est compris entre 1:4 et 4:1.

15. Procédé selon la revendication 14, caractérisé en ce que le rapport en volume de la phase aqueuse à la phase organique est compris entre 1:1 et 1:2.
